# EUROPEAN PATENT APPLICATION

(11) **EP 3 040 069 A1**
(43) Date of publication of application: **06.07.2016**
(21) Application number: 14817431.1
(22) Date of filing: 24.06.2014
(51) Int. Cl.: A61K 31/122, A61P 1/14

(54) **INTESTINAL FLORA IMPROVING COMPOSITION CONTAINING ASTAXANTHIN**

(30) Priority: 27.06.2013 JP 2013134704
(71) Applicant: The Doshisha, Kyoto 602-8580 (JP); Fuji Chemical Industry Co., Ltd., Nakaniikawa-gun, Toyama 930-0397 (JP)
(72) Inventor: YONEI, Yoshikazu, Kyotanabe-shi Kyoto 610-0394 (JP); TOMINAGA, Kumi, Nakaniikawa-gun Toyama 930-0397 (JP); YAMASHITA, Eiji, Nakaniikawa-gun Toyama 930-0397 (JP)
(74) Representative: Wunderlich, Rainer
(86) International application number: PCT/JP2014/066600
(87) International publication number: WO 2014/208511

(57) **Abstract**

An oral composition improves the balance of intestinal flora via oral intake. The oral composition that improves intestinal flora via oral intake includes astaxanthin as an active ingredient, and excludes a component that can form skin tissue, the composition improving the intestinal flora by reducing or suppressing either or both of an increase in gram-negative bacteria and an increase in bacteria that belong to the *Clostridium coccoides* group.

## Description

### TECHNICAL FIELD

The present invention relates to an oral composition that improves intestinal flora.

### BACKGROUND ART

Astaxanthin is a free radical-scavenging antioxidant. It is known that astaxanthin is absorbed in the small intestine when taken orally, and exhibits an antioxidant effect.

For example, one of the inventors of the present application has reported that astaxanthin enhances the antioxidant potential, decreases blood pressure, and improves constipation and a climacteric complaint (see Non-Patent Literature 1).

However, only about 5% of astaxanthin is absorbed in the small intestine (i.e., most of the astaxanthin remains in the intestine without being absorbed), and the fecal color changes to red due to astaxanthin.

The inventors of the invention conducted studies with regard to the effects of astaxanthin on intestinal flora, and completed the invention.

The term "intestinal flora" refers to an intestinal ecosystem that is formed by a number of types of resident bacteria.

Since ingested food and the like are broken up and absorbed in the intestine, the intestine is abundant in nutrients necessary for living organisms to grow, and holds a large number of resident bacteria as compared with other human/animal organs.

Such various bacteria compete for survival in the intestine, and establish a symbiotic relationship to form intestinal flora that keeps a constant (equilibrium) balance.

The term "intestinal flora" is also referred to as "gut flora".

Intestinal flora changes with aging or depending on the living environment, and affects the health condition or causes disease (e.g., cancer). It is important to keep healthy intestinal flora in order to maintain a young and healthy body.

Intestinal bacteria that form intestinal flora are generally classified as good bacteria that are useful for the host (e.g., human), bad bacteria that adversely affect the host (e.g., cause intestinal putrefaction), or opportunistic organisms that do not act as good bacteria or bad bacteria in a healthy state, but act as bad bacteria in an unhealthy state, or when the balance of intestinal flora has been lost.

In recent years, it has been pointed out that the balance of intestinal flora may deteriorate due to a high-fat diet, and such a high-fat diet has posed a social problem.

It is known that intestinal flora that has deteriorated in balance may be improved by the intake of dietary fiber that may promote digestive peristalsis, or the intake of an oligosaccharide that is consumed by good bacteria. Note that the effects of astaxanthin on intestinal flora have not been studied or reported.

Non-Patent Literature 1: Iwabayashi M, Fujioka N, Nomoto K, Miyazaki R, Takahashi H, Hibino S, Takahashi Y, Nishikawa K, Nishida M, Yonei Y., "Efficacy and safety of eight-week treatment with astaxanthin in individuals screened for increased oxidative stress burden", Anti-Aging Medicine 6: 15-21, 2009

### SUMMARY OF INVENTION

### TECHNICAL PROBLEM

An object of the invention is to provide a composition that improves the balance of intestinal flora via oral intake.

### SOLUTION TO PROBLEM

One aspect of the invention provides an intestinal flora-improving oral composition that improves intestinal flora via oral intake, the composition comprising astaxanthin as an active ingredient, and excluding a component that can form skin tissue, the composition improving the intestinal flora by reducing or suppressing either or both of an increase in gram-negative bacteria and an increase in bacteria that belong to the *Clostridium coccoides* group.

The intestinal flora-improving oral composition improves intestinal flora via oral intake, the composition comprising astaxanthin as an active ingredient, and excluding a component that can form skin tissue, the composition improving the intestinal flora by increasing bacteria that belong to the genus *Lactobacillus* and bacteria that belong to the genus *Streptococcus.*

The intestinal flora-improving oral compositions improve intestinal flora by reducing or suppressing growth of bad bacteria, increasing growth of good bacteria, reducing or suppressing an infection due to an opportunistic organism, reducing or suppressing an increase in gram-negative bacteria, reducing or suppressing an increase in bacteria that belong to the *Clostridium coccoides* group, and increasing bacteria that belong to the genus *Lactobacillus* and bacteria that belong to the genus *Streptococcus.*

The intestinal flora-improving oral compositions may improve intestinal flora by reducing or suppressing the disorder of intestinal flora, or improve intestinal flora by reducing or suppressing the growth of bad bacteria, and increasing the growth of good bacteria, or improve intestinal flora by reducing or suppressing an infection due to an opportunistic organism.

Another aspect of the invention provides use of an intestinal flora-improving oral composition that includes astaxanthin as an active ingredient for improving intestinal flora via oral intake.

The intestinal flora-improving oral compositions according to the invention do not include a component that can form skin tissue, such as low-molecular-weight collagen, dermatan sulfate, hyaluronic acid, chondroitin, and ceramide.

Typical examples of intestinal bacteria that act as good bacteria include bacteria that belong to the genus *Lactobacillus,* bacteria that belong to the genus *Streptococcus,* and bifidobacteria (bacteria that belong to the genus *Bifidobacterium*)*.*

### Bacteria that belong to the genus Lactobacillus (lactic acid bacteria)

*Lactobacillus* is a genus of gram-positive bacilli.

Bacteria that belong to the genus *Lactobacillus* produce only lactic acid (homolactic fermentation), or simultaneously produce lactic acid and a product other than lactic acid (heterolactic fermentation).

A large number of bacteria that belong to the genus *Lactobacillus* live in a human/animal digestive tract, and bacteria that belong to the genus *Lactobacillus* can be separated from feces.

Bacteria that belong to the genus *Lactobacillus* live in a wide variety of environments such as fermented food and an animal digestive tract.

The genus *Lactobacillus* includes 100 or more different bacterial species.

### Bacteria that belong to the genus Streptococcus (lactic acid bacteria)

*Streptococcus* is a genus of coccus gram-positive bacteria (eubacteria).

Bacteria that belong to the genus *Streptococcus* have a diameter of about 1 µm, and have a structure in which individual cells are regularly and linearly arranged.

Bacteria that belong to the genus *Streptococcus* differ from other coccus gram-positive bacteria in that bacteria that belong to the genus *Streptococcus* biochemically do not have catalase.

Bacteria that belong to the genus *Streptococcus* normally do not produce energy through respiration, and mainly obtain energy through lactic acid fermentation.

Bacteria that belong to the genus *Streptococcus* generally function as lactic acid bacteria inside the intestine.

Enterococcus was classified as the genus *Streptococcus,* but is classified as the family *Enterococcaceae.*

Bacteria that belong to the genus *Streptococcus* include *E. faecalis, E. faecium*, and the like, and live in the ileum, the cecum, and the large intestine.

### Bifidobacteria

The term "bifidobacteria" is a generic name for gram-positive obligately anaerobic bacilli that belong to the genus *Bifidobacterium* that belongs to the order *Bifidobacteriales* that belongs to the class *Actinobacteria.* Bifidobacteria live in the human/animal intestine.

*B. bifidum, B. breve, B. infantis* (classified as *B. longum* subsp. *infantis), B. longum,* and *B. adolescentis* are found in the human intestine.

Bifidobacteria decompose sugar to produce lactic acid and acetic acid, and improve the intestinal environment as good bacteria.

In recent years, it has been reported that bifidobacteria that live in the intestine have an influenza virus infection preventive effect and an allergy symptom (e.g., pollinosis)-relieving effect.

A large number of bifidobacteria are present in feces of a breast-fed child, and the number of bifidobacteria gradually decreases with aging or depending on the dietary environment.

Typical examples of bifidobacteria that act as bad bacteria include bacteria that belong to the genus *Clostridium.*

### Bacteria that belong to the genus Clostridium

*Clostridium* is a genus of eubacteria. Bacteria that belong to the genus *Clostridium* are gram-positive obligately anaerobic and spore-forming bacilli.

Bacteria that belong to the genus *Clostridium* are obligate anaerobes that live in an environment with a low oxygen concentration (e.g., soil or intestine), and cannot grow in the presence of oxygen.

Since obligate anaerobes normally do not have superoxide dismutase and catalase (i.e., antioxidative enzymes), obligate anaerobes are inactivated in the presence of oxygen. However, bacteria that belong to the genus *Clostridium* form a spore with high durability in the presence of oxygen, and rest for a long time.

Therefore, bacteria that belong to the genus *Clostridium* can survive in an environment in which other obligate anaerobes cannot survive.

*C. tetani, C. botulinum, C. perfringens,* histotoxic clostridia (e.g., *C. novyi* and *C. septicum), C. difficile,* and the like belong to the genus *Clostridium.*

*C. coccoides* and *C. leptum* among bacteria that belong to the genus *Clostridium* are predominantly present in the intestine, but *Clostridium perfringens* and *C. difficile* are also found in the intestine.

*Clostridium perfringens* is a bacterium that lives in the human/animal intestine. *Clostridium perfringens* may produce a toxin, and cause food poisoning.

Typical examples of bacteria that belong to opportunistic organisms include bacteria that belong to the genus *Bacteroides* and bacteria that belong to the genus *Prevotella.*

### Bacteria that belong to the genus Bacteroides

*Bacteroides* is a genus of gram-negative obligately anaerobic and non-spore-forming bacilli (e.g., *Bacteroides fragilis*).

A large number of bacteria that belong to the genus *Bacteroides* are present in the human/animal intestine, and ferment sugar to produce lactic acid, acetic acid, and the like.

Bacteria that belong to the genus *Bacteroides* are present in human stool in a number of 10 billion to 100 billion per gram.

Bacteria that belong to the genus *Bacteroides* basically do not cause disease, but may cause an opportunistic infection.

### Bacteria that belong to the genus Prevotella

*Prevotella* is a genus of gram-negative anaerobic bacilli that are found in the mouth and the intestine.

Most of the oral infections are caused by resident bacteria and other weakly pathogenic bacteria. However, an infection caused by weakly pathogenic bacteria may lead to an opportunistic infection.

Bacteria that belong to the genus *Prevotella* may cause an oral infection such as postextraction bacteremia, and may cause aspiration pneumonia that may be observed in older adults.

Most of the human anaerobic infections are caused by a combination of aerobic bacteria and anaerobic bacteria. Bacteria that belong to the genus *Prevotella* may cause such human anaerobic infections.

Astaxanthin that is used in connection with the invention is a carotenoid that is classified as a xanthophyll.

The IUPAC name of astaxanthin is 3,3'-dihydroxy-β,β-carotene-4,4'-dione. Three astaxanthin stereoisomers ((3R,3'R)-astaxanthin, (3R,3'S)-astaxanthin (meso-astaxanthin), and (3S,3'S)-astaxanthin) that differ in the positions of the hydroxyl groups (3-position and 3'-position) are known. There are cis-trans isomers depending on the conjugated double bond.

Any of these isomers may be used in connection with the invention.

Astaxanthin may be present in the form of a free compound, a monoester, and a diester.

Examples of natural astaxanthin include astaxanthin extracted from microalgae such as green algae (*Haematococcus*), yeast such as red yeast (*Phaffia*), the shell of an arthropod (e.g., prawn, krill, crab, and water flea), the internal organ and the gonad of a mollusk (e.g., squid and octopus), the skin of fish and shellfish, the petal of the genus *Amur amurensis* (e.g., *Adonis aestivalis*), α-proteobacteria (e.g., *Paracoccus* sp. N81106, *Brevundimonas* sp. SD212, and *Erythrobacter* sp. PC6), plants that belong to the genus *Gordonia* (e.g., *Gordonia* sp. KANMONKAZ-1129), *Labyrinthulea* (e.g., *Schizochytriuym* sp. KH105) (particularly *Thraustochytrium*), an astaxanthin-producing recombinant, and the like.

The intestinal flora-improving oral composition may be orally taken in the form of a supplement, health food, food with nutrient function claims, health function food (e.g., specified health food), special food, common food, a quasi drug, or a sports supplement. The intestinal flora-improving oral composition is preferably orally taken in the form of a supplement, a sports supplement, health function food, or special food from the viewpoint of ease of intake and ease of determination of the amount of intake. The intestinal flora-improving oral composition may be taken in the form of a solid (e.g., tablet, in-mouth rapidly-disintegrable tablet, capsule, granules, or minute granules) or a liquid (e.g., liquid, drink, syrup, or suspension).

The intestinal flora-improving oral composition may be mixed with a fibrous substance such as dietary fiber.

### ADVANTAGEOUS EFFECTS OF INVENTION

Resident bacteria that are specific to each individual live in the intestine, and form intestinal flora.

An equilibrium relationship is normally established between the intestinal flora and the host, and between the bacterial species that form the intestinal flora. However, when the equilibrium relationship is lost for some reason, microbial substitution or an opportunistic infection occurs, and the aging of the host, nourishment, the medicinal effect, physiology, the immunologic mechanism, and the onset of cancer are significantly affected.

It was found that the intake of astaxanthin controls the disorder of intestinal flora when a high-fat diet is fed to a mouse (described later).

It is considered that, when a human takes astaxanthin, the astaxanthin that remains in the intestine without being absorbed improves the disorder of intestinal flora caused by a high-fat diet.

### BRIEF DESCRIPTION OF DRAWINGS

FIG. 1 illustrates a change in body weight.
FIG. 2 illustrates a change in fecal weight.
FIG. 3 illustrates a change in DNA content (using the NanoDrop method).
FIG. 4 illustrates a change in the estimated copy number (%) of the *Lactobacillus* group (lactic acid bacteria) based on the total primer measured value.
FIG. 5 illustrates a change in the estimated copy number (%) of the *Streptococcus species* group (lactic acid bacteria) based on the total primer measured value.
FIG. 6 illustrates a change in the estimated copy number (%) of bifidobacteria based on the total primer measured value.
FIG. 7 illustrates a change in the estimated copy number (%) of the *Clostridium coccoides* group based on the total primer measured value.
FIG. 8 illustrates a change in the estimated copy number (%) of the *Clostridium leptum* group based on the total primer measured value.
FIG. 9 illustrates a change in the estimated copy number (%) of bacteria that belong to the genus *Bacteroides* based on the total primer measured value.
FIG. 10 illustrates a change in the estimated copy number (%) of bacteria that belong to the genus *Prevotella* based on the total primer measured value.

### DESCRIPTION OF EMBODIMENTS

A change in intestinal flora when a high-fat diet was fed to mice, and the effect of astaxanthin on intestinal flora were determined using a real-time PCR method.

### Preparation of ordinary diet and high-fat diet containing test substance

The test substance (astaxanthin) was added to an ordinary diet ("Labo MR Stock" manufactured by Nosan Corporation (Nishi-ku, Yokohama)) and a high-fat diet ("HFD-60" Oriental Yeast Co., Ltd. (Itabashi-ku, Tokyo)) so that the content thereof was 0.02%.

A commercially-available product "AstaReal Oil 50F" (manufactured by Fuji Chemical Industries Co., Ltd.) was used as astaxanthin.

### Animal feeding and assay method

Twenty 4-week-old ICR mice were respectively kept in different cages, and habituated for 1 week.

After completion of habituation, the weight of each mouse was measured, and the mice were divided into an ordinary diet group (group C), an ordinary diet+astaxanthin group (group CA), a high-fat diet group (group H), and a high-fat diet+astaxanthin group (group HA) (five mice per group).

The feces of the mice were collected over 24 hours on the final day of habituation (Day 0).

The diet was fed to each group from Day 1, and the weight of each mouse was measured once a week.

The ordinary diet was fed once a week, and the high-fat diet was fed every other day (the high-fat diet was returned to room temperature on the day before the feeding).

The feces of the mice were also collected from the thirteenth day to the fourteenth day (Day 14) and from the twenty-seventh day to the twenty-eighth day (Day 28).

The collected feces were stored at -20°C.

### Extraction of DNA

The feces collected on Day 0, Day 14, and Day 28 were thawed, weighed, and dried at room temperature overnight.

The dried feces were weighed on the next day, and powdered.

About 100 mg of the powdered feces was weighed on a micro test tube, and the DNA was extracted using a QIAampR DNA Stool Mini Kit (manufactured by Qiagen (Venlo, the Netherlands)).

The concentration of the extracted DNA solution was measured using the NanoDrop method.

### Real-time PCR

The DNA solution was 100-fold diluted (*Lactobacillus, Streptococcus, Clostridium coccoides,* and *Clostridium leptum*), and the final concentration was adjusted to 20 µg/µL (other targets). A real-time PCR assay was performed according to the protocol of a LightCycler (registered trademark) 480 SYBR Green I Master (Roche Diagnostics K.K. (Minato-ku, Tokyo)). The primer sequence was analyzed using the method of Matsuki et al. or Endo et al.

The real-time PCR assay was performed on *Bacteroides*, *Bifidobacterium* (bifidobacteria), *Prevotella, Lactobacillus, Streptococcus, Clostridium coccoides,* and *Clostridium leptum.*

### Statistical analysis

Each DNA content refers to "mean value±standard deviation".

The mean value of each group was analyzed by Tukey's multiple comparison test.

Regarding a comparison between the ordinary diet and the high-fat diet, the bacterial count change ratio (Day 0, Day 14, and Day 28) of the ordinary diet group (group C and group CA) and the high-fat diet group (group H and group HA) was analyzed by Mann-Whitney's U test (two-sided test).

Regarding a comparison as to the presence or absence of astaxanthin, the bacterial count change ratio was analyzed by Mann-Whitney's U test (one-sided test).

The statistical analysis was performed using SPSS for Windows Ver.15.0 (registered trademark) (manufactured by SPSS Inc. (Chicago, IL, USA)).

The measurement results and the analysis results are discussed below.

### Change in body weight and properties of feces

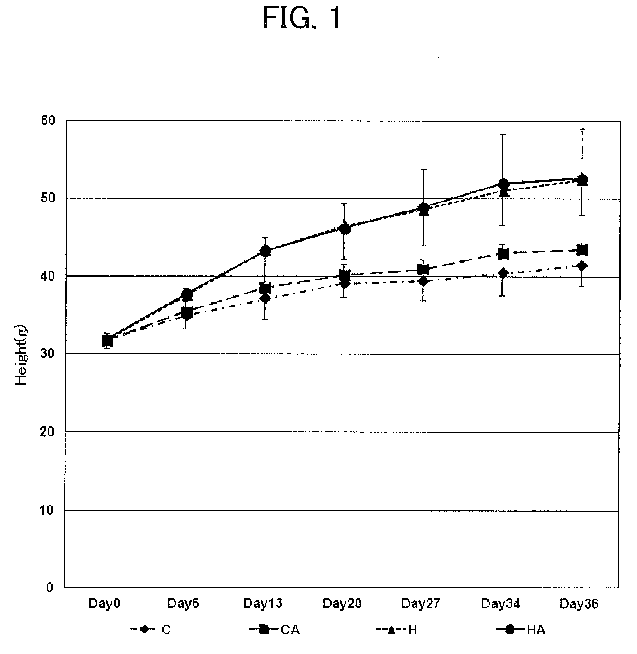

FIG. 1 illustrates a change in body weight.

The group H showed a significant increase in weight as compared with the group C (p<0.01, p<0.05).

The group HA showed a significant increase in weight as compared with the group CA (p<0.05).

No significant difference was observed between the groups to which astaxanthin was fed (group CA and group HA) and the groups to which astaxanthin was not fed (group C and group H).

The color of the feces of the group C was dark green, the color of the feces of the group CA was red brown, the color of the feces of the group H was light greenish gray, and the color of the feces of the group HA was red to orange.

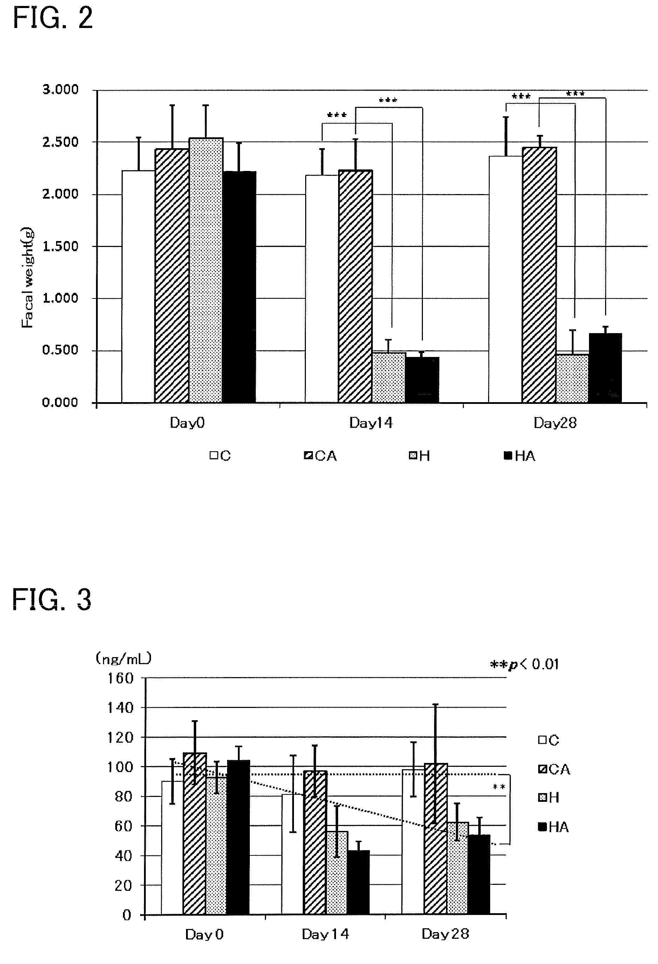

As illustrated in FIG. 2, the fecal weight of the group H on Day 14 and Day 28 was significantly lower than that of the group C, and the fecal weight of the group HA on Day 14 and Day 28 was significantly lower than that of the group CA.

### DNA content

FIG. 3 illustrates a change in the content of DNA extracted from the feces using the NanoDrop method (mean value (Day 0, Day 14, and Day 28)).

The DNA content of the group C was 89.8±21.5 ng/µL, the DNA content of the group CA was 102.7±28.5 ng/µL, the DNA content of the group H was 70.2±21.2 ng/µL, and the DNA content of the group HA was 66.9±28.4 ng/µL. The high-fat diet group (group H+group HA) showed a decrease in DNA content as compared with the ordinary diet group (group C+group CA) (p=0.001).

A change in respective bacteria is described below.

### Lactobacillus group (lactic acid bacteria)

The bacterial count of the *Lactobacillus* group was less than 3% based on the total bacterial count.

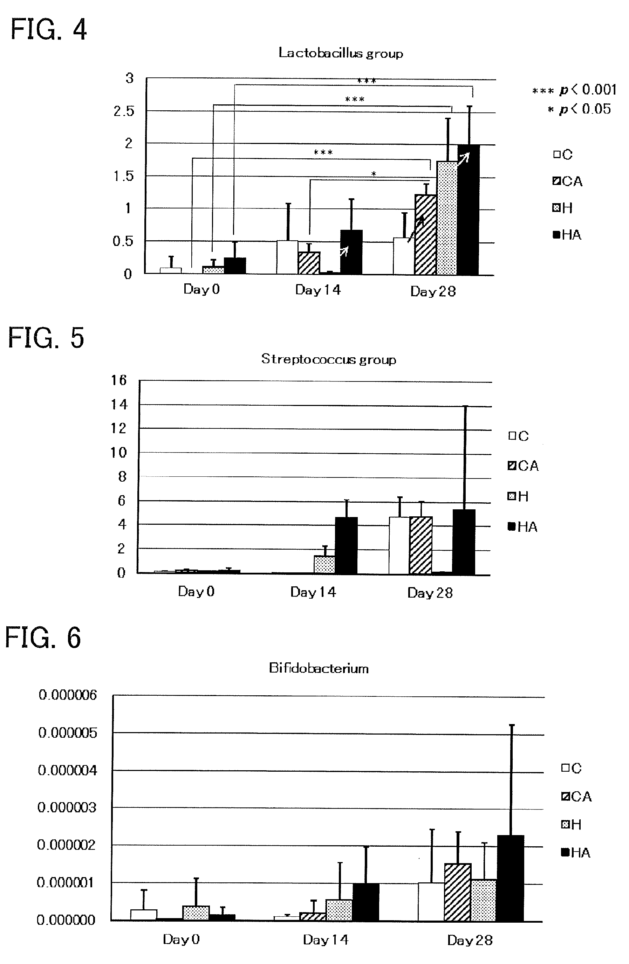

FIG. 4 illustrates a change in the bacterial count of the *Lactobacillus* group.

The bacterial count of the *Lactobacillus* group significantly increased due to the high-fat diet (p=0.004).

The ordinary diet group (group C and group HA) showed an increase in the bacterial count of the *Lactobacillus* group with the passage of time (see Day 14 and Day 28).

No significant difference in the rate of increase in the bacterial count of the *Lactobacillus* group was observed between the ordinary diet group and the high-fat diet group (p=0.069).

The rate of increase in the bacterial count of the *Lactobacillus* group increased due to the addition of astaxanthin (p=0.031).

### Streptococcus group (lactic acid bacteria)

The bacterial count of the *Streptococcus* group was less than 16% based on the total bacterial count.

FIG. 5 illustrates a change in the bacterial count of the *Streptococcus* group.

The bacterial count of the *Streptococcus* group significantly increased due to the ordinary diet, and a change in the bacterial count of the *Streptococcus* group due to the high-fat diet was small (p=0.028).

The high-fat diet group showed a significantly low rate of increase in the bacterial count of the *Streptococcus* group as compared with the ordinary diet group (p=0.008).

The bacterial count of the *Streptococcus* group of the group HA (to which astaxanthin was fed) on Day 28 was comparable to those of the ordinary diet group (group C and group CA).

A significant difference was observed between the high-fat diet groups due to the addition of astaxanthin (p=0.044).

### Bifidobacteria

The bacterial count of bifidobacteria was less than 0.000006% based on the total bacterial count.

FIG. 6 illustrates a change in the bacterial count of bifidobacteria.

Since the bacterial count of bifidobacteria is very small, the bacterial count of bifidobacteria is illustrated as a reference value.

### Clostridium coccoides group

The bacterial count of the *Clostridium coccoides* group was less than 1.4% based on the total bacterial count.

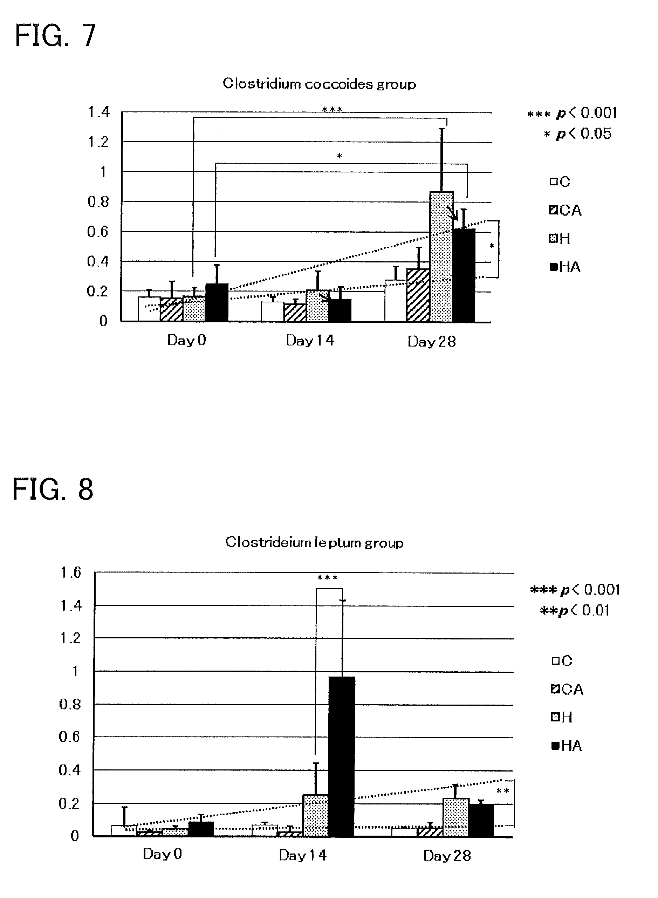

FIG. 7 illustrates a change in the bacterial count of the *Clostridium coccoides* group.

The bacterial count of the *Clostridium coccoides* group significantly increased due to the high-fat diet (p=0.016).

The high-fat diet group showed a high rate of increase in the bacterial count of the *Clostridium coccoides* group as compared with the ordinary diet group (p=0.012).

When the high-fat diet was fed to the mice, the rate of increase in the bacterial count of the *Clostridium coccoides* group decreased due to the addition of astaxanthin (p=0.029).

### Clostridium leptum group

The bacterial count of the *Clostridium leptum* group was less than 1.6% based on the total bacterial count.

FIG. 8 illustrates a change in the bacterial count of the *Clostridium leptum* group.

The bacterial count of the *Clostridium leptum* group significantly increased due to the high-fat diet (p=0.017).

The high-fat diet group showed a high rate of increase in the bacterial count of the *Clostridium leptum* group as compared with the ordinary diet group (p=0.002).

The group HA showed a temporary increase in the bacterial count of the *Clostridium leptum* group on Day 14 (p<0.001 with respect to the group H), but no significant difference in the bacterial count of the *Clostridium leptum* group was observed between the group H and the group HA on Day 28.

### Bacteria that belong to the genus Bacteroides

The bacterial count of bacteria that belong to the genus *Bacteroides* was less than 0.003% based on the total bacterial count.

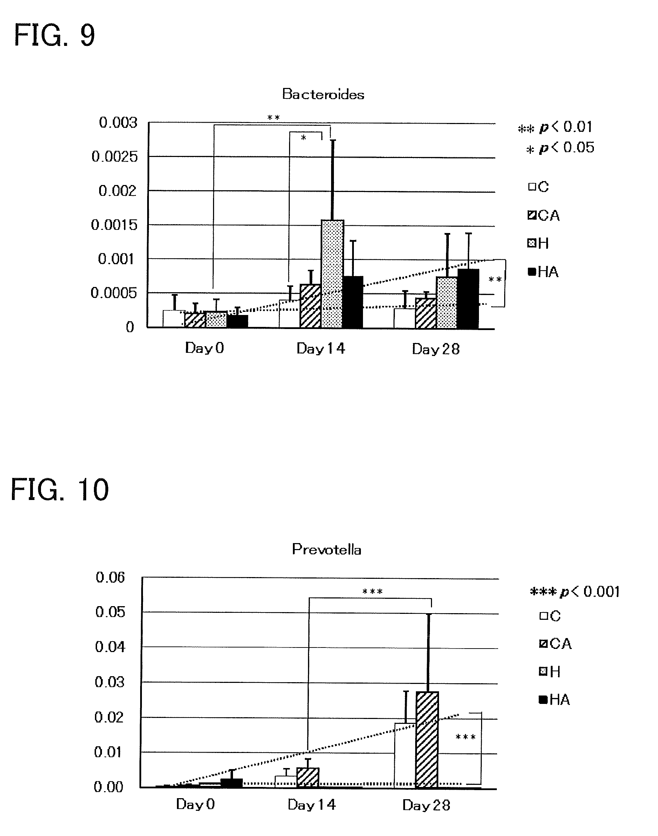

FIG. 9 illustrates a change in the bacterial count of bacteria that belong to the genus *Bacteroides.*

The bacterial count of bacteria that belong to the genus *Bacteroides* significantly increased due to the high-fat diet (p=0.006).

The group H showed a temporary increase in the bacterial count of bacteria that belong to the genus *Bacteroides* on Day 14 (p<0.01 with respect to Day 0, p<0.05 with respect to the group C), but the bacterial count of bacteria that belong to the genus *Bacteroides* returned on Day 28 to a level comparable to that on Day 0.

The group HA did not show such a temporary increase in the bacterial count of bacteria that belong to the genus *Bacteroides.*

The high-fat diet group showed a high rate of increase in the bacterial count of bacteria that belong to the genus *Bacteroides* as compared with the ordinary diet group (p=0.002).

### Bacteria that belong to the genus Prevotella

The bacterial count of bacteria that belong to the genus *Prevotella* was less than 0.06% based on the total bacterial count.

FIG. 10 illustrates a change in the bacterial count of bacteria that belong to the genus *Prevotella.*

The bacterial count of bacteria that belong to the genus *Prevotella* significantly decreased due to the high-fat diet (p<0.001).

The ordinary diet group showed a high rate of increase in the bacterial count of bacteria that belong to the genus *Prevotella* as compared with the high-fat diet group (p<0.001).

The following were confirmed from the above results.

Astaxanthin suppressed a temporary increase in the bacterial count of bacteria that belong to the genus *Bacteroides* (gram-negative bacteria) due to the high-fat diet (see the graph illustrated in FIG. 9).

Specifically, while the group H showed a significant increase in the bacterial count of bacteria that belong to the genus *Bactenoides* on Day 14, the group HA did not show a significant increase in the bacterial count of bacteria that belong to the genus *Bacteroides* on Day 14.

The addition of astaxanthin increased the bacterial count of the *Lactobacillus* group (lactic acid bacteria) (see the graph illustrated in FIG. 4). This is clear from a comparison between the group C and the group CA and a comparison between the group H and the group HA.

Likewise, the addition of astaxanthin to the high-fat diet increased the bacterial count of the *Streptococcus* group (lactic acid bacteria) (see the graph illustrated in FIG. 5). This is clear from a comparison between the group H and the group HA on Day 28.

It was thus confirmed that the intake of astaxanthin improves intestinal flora (i.e., suppresses the runaway of gram-negative bacteria, and increases the bacterial count of lactic acid bacteria).

Astaxanthin also suppressed the growth of the *Clostridium coccoides* group (bad bacteria) (see the graph illustrated in FIG. 7). This is clear from a comparison between the group H and the group HA (to which the high-fat diet was fed) on Day 14 and Day 28.

### INDUSTRIAL APPLICABILITY

The composition and the like according to the invention are useful for improving intestinal flora.

## Claims

1. An intestinal flora-improving oral composition that improves intestinal flora via oral intake, the composition comprising astaxanthin as an active ingredient, and excluding a component that can form skin tissue, the composition improving the intestinal flora by reducing or suppressing either or both of an increase in gram-negative bacteria and an increase in bacteria that belong to the *Clostridium coccoides* group.

2. An intestinal flora-improving oral composition that improves intestinal flora via oral intake, the composition comprising astaxanthin as an active ingredient, and excluding a component that can form skin tissue, the composition improving the intestinal flora by increasing bacteria that belong to the genus *Lactobacillus* and bacteria that belong to the genus *Streptococcus.*

3. An intestinal flora-improving oral composition that improves intestinal flora via oral intake, the composition comprising astaxanthin as an active ingredient, and excluding a component that can form skin tissue, the composition improving the intestinal flora by reducing or suppressing growth of bad bacteria, increasing growth of good bacteria, reducing or suppressing an infection due to an opportunistic organism, reducing or suppressing an increase in gram-negative bacteria, reducing or suppressing an increase in bacteria that belong to the *Clostridium coccoides* group, and increasing bacteria that belong to the genus *Lactobacillus* and bacteria that belong to the genus *Streptococcus.*

4. Use of the intestinal flora-improving oral composition as defined in any one of claims 1 to 3 for improving intestinal flora via oral intake.
